# FASCICULE DE BREVET EUROPEEN

(11) **EP 2 358 751 B1**
(45) Date de publication et mention de la délivrance du brevet: **11.05.2016**
(21) Numéro de dépôt: 09793553.0
(22) Date de dépôt: 17.11.2009
(51) Int. Cl.: C07K 14/705, C12N 15/12, A61K 48/00, C12Q 1/68, G01N 33/68, A61K 38/17

(54) **PROTÉINE CD20 TRONQUÉE, DELTACD20**
ABGESCHNITTENES PROTEIN CD20 , DELTA CD20
TRUNCATED CD20 PROTEIN , DELTA CD20

(30) Priorité: 18.11.2008 FR 0806444
(43) Date de publication de la demande: 24.08.2011
(73) Titulaire: Etablissement Français Du Sang (EFS), 93218 La Plaine Saint Denis (FR)
(72) Inventeur: FERRAND, Christophe, F-39700 Dampierre (FR); DESCHAMPS, Marina, F-25000 Besancon (FR); HENRY, Carole, F-25000 Besancon (FR); TIBERGHIEN, Pierre, F-75009 Paris (FR); BORG, Christophe, F-25115 Pouilley les Vignes (FR); ROHRLICH, Pierre-Simon, F-25000 Besancon (FR)
(74) Mandataire: Oudin, Stéphane
(86) Numéro de dépôt international: PCT/FR2009/001315
(87) Numéro de publication internationale: WO 2010/058097

(56) Documents cités:
- DATABASE BIOSIS [Online] BIOSCIENCES INFORMATION SERVICE, PHILADELPHIA, PA, US; novembre 2008 (2008-11), DESCHAMPS MARINA ET AL: "A New Human CD20 Spliced mRNA as a Potential Molecular Marker for the Follow-up of B-Cell Malignancies." XP002570093 Database accession no. PREV200900258179
- BLOOD, vol. 112, no. 11, 16 novembre 2008 (2008-11-16), page 526, XP002570094 50TH ANNUAL MEETING OF THE AMERICAN- SOCIETY-OF-HEMATOLOGY; SAN FRANCISCO, CA, USA; DECEMBER 06 -09, 2008 ISSN: 0006-4971
- DATABASE EMBL [Online] 24 juillet 2008 (2008-07-24), "Homo sapiens cDNA FLJ51650 complete cds, moderately similar to B-lymphocyte antigen CD20." XP002531748 extrait de EBI accession no. EMBL:AK300025 Database accession no. AK300025
- LIANG Y ET AL: "STRUCTURAL ORGANIZATION OF THE HUMAN MS4A GENE CLUSTER ON CHROMOSOME 11q12" IMMUNOGENETICS, SPRINGER VERLAG, BERLIN, DE, vol. 53, no. 5, 1 juillet 2001 (2001-07-01), pages 357-368, XP009053461 ISSN: 0093-7711
- CRAGG M S ET AL: "The biology of CD20 and its potential as a target for mAb therapy" CURRENT DIRECTIONS IN AUTOIMMUNITY, KARGER, BASEL, CH, 1 janvier 2005 (2005-01-01), pages 140-174, XP008087727 ISSN: 1422-2132
- EINFELD D A ET AL: "Molecular cloning of the human B cell CD20 receptor predicts a hydrophobic protein with multiple transmembrane domains" EMBO JOURNAL, OXFORD UNIVERSITY PRESS, SURREY, GB, vol. 7, no. 3, 1 janvier 1988 (1988-01-01) , pages 711-717, XP002997280 ISSN: 0261-4189
- STAMENKOVIC I ET AL: "ANALYSIS OF TWO CDNA CLONES ENCODING THE B LYMPHOCYTE ANTIGEN CD20 (B1, BP35), A TYPE III INTEGRAL MEMBRANE PROTEIN" THE JOURNAL OF EXPERIMENTAL MEDICINE, ROCKEFELLER UNIVERSITY PRESS, UNITED STATES, vol. 167, no. 6, 1 juin 1988 (1988-06-01), pages 1975-1980, XP009065525 ISSN: 0022-1007

### Listage de séquences

<110> ETABLISSEMENT FRANÇAIS DU SANG (EFS)
<120> Protéine CD20 tronquée, DeltaCD20
<130> EFS001-WO-1
<150> FR 08/06444
   <151> 2008-11-18
<160> 17
<170> Patent In version 3.5
<210> 1
   <211> 393
   <212> DNA
   <213> Homo sapiens
<400> 1
   atgacaacac ccagaaattc agtaaatggg actttcccgg cagagccaat gaaaggccct     60
   attgctatgc aatctggtcc aaaaccactc ttcaggagga tgtcttcact ggaacttgta    120
   atagctggca tcgttgagaa tgaatggaaa agaacgtgct ccagacccaa atctaacata    180
   gttctcctgt cagcagaaga aaaaaaagaa cagactattg aaataaaaga agaagtggtt    240
   gggctaactg aaacatcttc ccaaccaaag aatgaagaag acattgaaat tattccaatc    300
   caagaagagg aagaagaaga aacagagacg aactttccag aacctcccca agatcaggaa    360
   tcctcaccaa tagaaaatga cagctctcct taa                                 393
<210> 2
   <211> 130
   <212> PRT
   <213> Homo sapiens
<400> 2
   Met Thr Thr Pro Arg Asn Ser Val Asn Gly Thr Phe Pro Ala Glu Pro
   1               5                   10                  15
   Met Lys Gly Pro Ile Ala Met Gln Ser Gly Pro Lys Pro Leu Phe Arg
               20                  25                  30
   Arg Met Ser Ser Leu Glu Leu Val Ile Ala Gly Ile Val Glu Asn Glu
           35                  40                  45
   Trp Lys Arg Thr Cys Ser Arg Pro Lys Ser Asn Ile Val Leu Leu Ser
       50                  55                  60
   Ala Glu Glu Lys Lys Glu Gln Thr Ile Glu Ile Lys Glu Glu Val Val
   65                  70                  75                  80
   Gly Leu Thr Glu Thr Ser Ser Gln Pro Lys Asn Glu Glu Asp Ile Glu
                   85                  90                  95
   Ile Ile Pro Ile Gln Glu Glu Glu Glu Glu Glu Thr Glu Thr Asn Phe
               100                 105                 110
   Pro Glu Pro Pro Gln Asp Gln Glu Ser Ser Pro Ile Glu Asn Asp Ser
           115                 120                 125
   Ser Pro
       130
<210> 3
   <211> 1915
   <212> DNA
   <213> Homo sapiens
<400> 3
   agcgatttca tcttcaggcc tggactacac cactcaccct cccagtgtgc ttgagaaaca 60
   aactgcaccc actgaactcc gcagctagca tccaaatcag cccttgagat ttgaggcctt 120
   ggagactcag atcctgaaca agagagaaca aaatctctac tttgatggaa cttccattct 180
   gtggggaaga gactgacaat aagcaattaa ataaataaga actcagcagt aggccttgcc 240
   tcagatccaa ggtcactcgg aagaggccat gtctaccctc aatgacactc atggaggaaa 300
   tgctgagaga agcattcaga tgcatgacac aaggtaagac tgccaaaaat cttgttcttg 360
   ctctcctcat tttgttattt gttttatttt taggagtttt gagagcaaaa tgacaacacc 420
   cagaaattca gtaaatggga ctttcccggc agagccaatg aaaggcccta ttgctatgca 480
   atctggtcca aaaccactct tcaggaggat gtcttcactg gtgggcccca cgcaaagctt 540
   cttcatgagg gaatctaaga ctttgggggc tgtccagatt atgaatgggc tcttccacat 600
   tgccctgggg ggtcttctga tgatcccagc agggatctat gcacccatct gtgtgactgt 660
   gtggtaccct ctctggggag gcattatgta tattatttcc ggatcactcc tggcagcaac 720
   ggagaaaaac tccaggaagt gtttggtcaa aggaaaaatg ataatgaatt cattgagcct 780
   ctttgctgcc atttctggaa tgattctttc aatcatggac atacttaata ttaaaatttc 840
   ccatttttta aaaatggaga gtctgaattt tattagagct cacacaccat atattaacat 900
   atacaactgt gaaccagcta atccctctga gaaaaactcc ccatctaccc aatactgtta 960
   cagcatacaa tctctgttct tgggcatttt gtcagtgatg ctgatctttg ccttcttcca 1020
   ggaacttgta atagctggca tcgttgagaa tgaatggaaa agaacgtgct ccagacccaa 1080
   atctaacata gttctcctgt cagcagaaga aaaaaaagaa cagactattg aaataaaaga 1140
   agaagtggtt gggctaactg aaacatcttc ccaaccaaag aatgaagaag acattgaaat 1200
   tattccaatc caagaagagg aagaagaaga aacagagacg aactttccag aacctcccca 1260
   agatcaggaa tcctcaccaa tagaaaatga cagctctcct taagtgattt cttctgtttt 1320
   ctgtttcctt ttttaaacat tagtgttcat agcttccaag agacatgctg actttcattt 1380
   cttgaggtac tctgcacata cgcaccacat ctctatctgg cctttgcatg gagtgaccat 1440
   agctccttct ctcttacatt gaatgtagag aatgtagcca ttgtagcagc ttgtgttgtc 1500
   acgcttcttc ttttgagcaa ctttcttaca ctgaagaaag gcagaatgag tgcttcagaa 1560
   tgtgatttcc tactaacctg ttccttggat aggcttttta gtatagtatt tttttttgtc 1620
   attttctcca tcaacaacca gggagactgc acctgatgga aaagatatat gactgcttca 1680
   tgacattcct aaactatctt ttttttattc cacatctacg tttttggtgg agtccctttt 1740
   gcatcattgt tttaaggatg ataaaaaaaa ataacaacta gggacaatac agaacccatt 1800
   ccatttatct ttctacaggg ctgacattgt ggcacattct tagagttacc acaccccatg 1860
   agggaagctc taaatagcca acacccatct gttttttgta aaaacagcat agctt 1915
<210> 4
   <211> 297
   <212> PRT
   <213> Homo sapiens
<400> 4
   Met Thr Thr Pro Arg Asn Ser Val Asn Gly Thr Phe Pro Ala Glu Pro
   1               5                   10                  15
   Met Lys Gly Pro Ile Ala Met Gln Ser Gly Pro Lys Pro Leu Phe Arg
               20                  25                  30
   Arg Met Ser Ser Leu Val Gly Pro Thr Gln Ser Phe Phe Met Arg Glu
           35                  40                  45
   Ser Lys Thr Leu Gly Ala Val Gln Ile Met Asn Gly Leu Phe His Ile
       50                  55                  60
   Ala Leu Gly Gly Leu Leu Met Ile Pro Ala Gly Ile Tyr Ala Pro Ile
   65                  70                  75                  80
   Cys Val Thr Val Trp Tyr Pro Leu Trp Gly Gly Ile Met Tyr Ile Ile
                   85                  90                  95
   Ser Gly Ser Leu Leu Ala Ala Thr Glu Lys Asn Ser Arg Lys Cys Leu
               100                 105                 110
   Val Lys Gly Lys Met Ile Met Asn Ser Leu Ser Leu Phe Ala Ala Ile
           115                 120                 125
   Ser Gly Met Ile Leu Ser Ile Met Asp Ile Leu Asn Ile Lys Ile Ser
       130                 135                 140
   His Phe Leu Lys Met Glu Ser Leu Asn Phe Ile Arg Ala His Thr Pro
   145                 150                 155                 160
   Tyr Ile Asn Ile Tyr Asn Cys Glu Pro Ala Asn Pro Ser Glu Lys Asn
                   165                 170                 175
   Ser Pro Ser Thr Gln Tyr Cys Tyr Ser Ile Gln Ser Leu Phe Leu Gly
               180                 185                 190
   Ile Leu Ser Val Met Leu Ile Phe Ala Phe Phe Gln Glu Leu Val Ile
           195                 200                 205
   Ala Gly Ile Val Glu Asn Glu Trp Lys Arg Thr Cys Ser Arg Pro Lys
   210 215 220
   Ser Asn Ile Val Leu Leu Ser Ala Glu Glu Lys Lys Glu Gln Thr Ile
   225 230 235 240
   Glu Ile Lys Glu Glu Val Val Gly Leu Thr Glu Thr Ser Ser Gln Pro
   245 250 255
   Lys Asn Glu Glu Asp Ile Glu Ile Ile Pro Ile Gln Glu Glu Glu Glu
   260 265 270
   Glu Glu Thr Glu Thr Asn Phe Pro Glu Pro Pro Gln Asp Gln Glu Ser
   275 280 285
   Ser Pro Ile Glu Asn Asp Ser Ser Pro
   290 295
<210> 5
   <211> 893
   <212> DNA
   <213> Artificial Sequence
<220>
   <223> gene artificiellement muté, codant le CD20, ne contenant plus de site accepteur pour un épissage alternatif
<400> 5
   atgacaacac ccagaaattc agtaaatggg actttcccgg cagagccaat gaaaggccct 60
   attgctatgc aatctggtcc aaaaccactc ttcaggagga tgtcttcact ggtgggcccc 120
   acgcaaagct tcttcatgag ggaatctaag actttggggg ctgtccagat tatgaatggg 180
   ctcttccaca ttgccctggg gggtcttctg atgatcccag cagggatcta tgcacccatc 240
   tgtgtgactg tgtggtaccc tctctgggga ggcattatgt atattatttc cggatcactc 300
   ctggcagcaa cggagaaaaa ctccaggaag tgtttggtca aaggaaaaat gataatgaat 360
   tcattgagcc tctttgctgc catttctgga atgattcttt caatcatgga catacttaat 420
   attaaaattt cccatttttt aaaaatggag agtctgaatt ttattagagc tcacacacca 480
   tatattaaca tatacaactg tgaaccagct aatccctctg agaaaaactc cccatctacc 540
   caatactgtt acagcataca atctctgttc ttgggcattt tgtcagtgat gctgatcttt 600
   gccttcttcc aagaacttgt aatagctggc atcgttgaga atgaatggaa aagaacgtgc 660
   tccagaccca aatctaacat agttctcctg tcagcagaag aaaaaaaaga acagactatt 720
   gaaataaaag aagaagtggt tgggctaact gaaacatctt cccaaccaaa gaatgaagaa 780
   gacattgaaa ttatccaatc caagaagagg aagaagaaga aacagagacg aactttccag 840
   aacctcccca agatcaggaa tcctcaccaa tagaaaatga cagctctcct taa 893
<210> 6
   <211> 9
   <212> PRT
   <213> Artificial Sequence
<220>
   <223> peptide synthetique
<400> 6
   Leu Phe Arg Arg Met Ser Ser Leu Glu
   1               5
<210> 7
   <211> 9
   <212> PRT
   <213> Artificial Sequence
<220>
   <223> peptide synthetique
<400> 7
   Phe Arg Arg Met Ser Ser Leu Glu Leu
   1               5
<210> 8
   <211> 9
   <212> PRT
   <213> Artificial Sequence
<220>
   <223> peptide synthetique
<400> 8
   Arg Arg Met Ser Ser Leu Glu Leu Val
   1               5
<210> 9
   <211> 9
   <212> PRT
   <213> Artificial Sequence
<220>
   <223> peptide synthétique
<400> 9
   Arg Met Ser Ser Leu Glu Leu Val Ile
   1               5
<210> 10
   <211> 9
   <212> PRT
   <213> Artificial Sequence
<220>
   <223> peptide synthetique
<400> 10
   Met Ser Ser Leu Glu Leu Val Ile Ala
   1 5
<210> 11
   <211> 9
   <212> PRT
   <213> Artificial Sequence
<220>
   <223> peptide synthetique
<400> 11
   Ser Ser Leu Glu Leu Val Ile Ala Gly
   1 5
<210> 12
   <211> 9
   <212> PRT
   <213> Artificial Sequence
<220>
   <223> peptide synthetique
<400> 12
   Ser Leu Glu Leu Val Ile Ala Gly Ile
   1 5
<210> 13
   <211> 9
   <212> PRT
   <213> Artificial Sequence
<220>
   <223> peptide synthetique
<400> 13
   Leu Glu Leu Val Ile Ala Gly Ile Val
   1 5
<210> 14
   <211> 18
   <212> DNA
   <213> Artificial Sequence
<220>
   <223> oligonucleotide sens specifique du deltaCD20
<400> 14
   gatgtcttca ctggaact 18
<210> 15
   <211> 21
   <212> DNA
   <213> Artificial Sequence
<220>
   <223> oligonucleotide antisens specifique du CD20 humain
<400> 15
   ttaaggagac tgtcattttc t 21
<210> 16
   <211> 21
   <212> DNA
   <213> Artificial Sequence
<220>
   <223> oligonucleotide sns specifique des formes deltaCD20 et wtCD20
<400> 16
   gagccaatga aaggccctat t 21
<210> 17
   <211> 20
   <212> DNA
   <213> Artificial Sequence
<220>
   <223> oligonucleotide antisens specifique du delta CD20
<400> 17
   agctattaca agttccagtg 20

## Description

La présente invention concerne notamment une protéine issue d'un épissage alternatif du gène codant le CD20, les séquences d'acides nucléiques codant la protéine selon l'invention, une forme mutée du gène codant CD20 ainsi que des médicaments, des outils diagnostiques, des méthodes diagnostiques et des méthodes thérapeutiques utilisant la protéine et les séquences d'acides nucléiques selon l'invention.

La protéine CD20 exprimée sur les lymphocytes B, est codée par un gène appartenant à une famille localisée sur le chromosome 11 en région q12. Cette région définit un cluster de gènes nommé MS4A (pour Membrane Spanning 4 domain Subfamily A), avec 12 sous groupes désignés de MS4A1 à MS4A12. Ce cluster de gènes, s'étend sur 600Kb. La totalité du gène CD20 couvre 41,17 Kb repartit en 8 exons séparés par 7 introns.

L'ARN pré-messager CD20 a une taille de 14.95 Kb avec une séquence 5' non traduite (5'UTR) de 416pb. La région 3'UTR est de 2291pb suivi par une extension polyA et correspondrait à une séquence de régulation. La séquence code potentiellement pour une protéine de 297 acides aminés (AA) pour un poids moléculaire prédictif de 33.0 KDa. Il a été identifié différents site d'épissage dans la région 5'UTR, plus précisément dans l'exon 1, générant trois formes de transcrits de tailles différentes variant de 2,8, 2,6 et 3,4 Kb.

Les différentes formes de transcrits codent pour la protéine CD20 d'un poids moléculaire de 33KD; cependant, par Western blot et immunoprécipitation, il a été montré la présence de 3 isoformes de 33, 34.5 et 36KD qui ne correspondent pas aux variants épissés mais à des modifications post-transcriptionnelles, par phosphorylation. Cette protéine est constituée d'une région très hydrophobe, de 4 segments transmembranaires (AA 68 à 84), délimitant un domaine extracellulaire (AA codés par l'exon VI) et un domaine intracellulaire (AA codés par l'exon III, V, VII et VIII).

Bien que très utilisé comme marqueur des lymphocytes B du sang périphérique, dans les techniques de caractérisation de population lymphocytaire ou encore en diagnostique de certaines pathologies ou d'hémopathies B, la fonction de la protéine CD20 est encore mal connue à ce jour. Cependant la structure protéique de la molécule CD20 humaine ou murine est similaire à d'autres protéines comme la rhodopsine, les protéines des jonctions Gap ou certains récepteurs adrénergiques, toutes impliquées dans la transduction du signal, ce qui laisse envisager un rôle similaire de cette protéine. La portion intracellulaire de cette protéine comporte de nombreuses séquences de phosphorylation et elle est associée à des tyrosines kinases de la famille des src (Fyn, Lyn, Lck)

Des études fonctionnelles in vitro et sur des modèles murins Knock-Out (KO) pour CD20, ont révélé que cette protéine était impliquée dans le transport inter-membranaire du Ca++. La liaison d'un Ac anti-CD20 à cette molécule induit également une augmentation des oncogènes c-Myc et B-Myb, une augmentation de la phosphorylation de protéines intracellulaires, une augmentation de CD18, CD58 et des molécules MHC de classe II ainsi qu'une activation de tyrosine kinase induisant une adhésion des cellules B. Ces fonctions attribuées à la protéine CD20 demeurent controversées, car le développement et la fonction de cellules B dans un modèle murin KO pour CD20 ont été rapportés comme normaux et ne présentent pas d'anomalies phénotypiques particulières.

Une autre fonction a été attribuée à la protéine CD20 qui est la régulation du cycle cellulaire dans la différenciation des lymphocytes B (LB) et leur activation/maturation en plasmocytes. Dans l'ontogenèse des lymphocytes B, CD20 est exprimé en grande quantité à la surface des cellules pré-B (absent sur les pro-B), après le réarrangement des gènes codant pour les chaînes lourdes des Ig, avec une expression membranaire persistante jusqu'au stade terminale B matures. Le CD20 n'est pas exprimé sur les cellules souches hématopoïétiques, sur les cellules pro-B, et les plasmocytes, sauf pour un petit contingent de cellules, dans certaines circonstances pathologiques, qui pourraient correspondrent à des plasmoblastes. Enfin rappelons que le ligand de CD20 n'est pas connu, ce qui rend également difficile la détermination de sa fonction.

L'expression à la surface des lymphocytes B permet la caractérisation de cette population en cytométrie de flux ou l'utilisation dans les techniques de purifications immuno-magnétiques. L'expression de la molécule CD20 sur la majorité des cellules B, impliquées dans les pathologies malignes en fait une cible thérapeutique de choix pour plusieurs raisons :
- Marqueur présent sur les LB et absent sur les cellules souches et les plasmocytes
- Elle est exprimée en grande quantité à la surface cellulaire
- Elle n'est pas secrétée ou libérée dans la circulation après protéolyse
- Après fixation de l'anti-CD20, le complexe CD20/Ac n'est pas internalisé

Le Rituximab(Rx, nom commercial : Mabthéra™) est un anticorps chimérique murin humanisé contre l'antigène CD20. Il est actif contre les cellules malignes présentant l'antigène CD20, c'est-à-dire dans les lymphomes folliculaires de stade III-IV et dans les lymphomes non-hodgkinien agressifs diffus à grandes cellules B, CD20 positifs. Il est également utilisé, associé ou non à de la chimiothérapie, et à titre plus expérimental dans d'autres pathologies, comme par exemple certaines maladies auto-immunes, telles que le lupus ou la polyarthrite Rhumatoïde.

La portion Fc de l'IgH humaine a été sélectionnée pour sa capacité à fixer le complément et entraîner une cytotoxicité de type ADCC (Antibody Dependant Cell Mediated. Les facteurs influençant sont efficacité sont divers : densité d'expression en surface de CD20, diffusion de l'anticorps, capture de l'anticorps anti-CD20 thérapeutique, liaison anticorps/cible, polymorphisme du récepteur FcgR3.

La présente invention repose sur la découverte d'un épissage alternatif du gène codant le CD20 conduisant à l'expression d'une forme tronquée de CD20. Ce polypeptide (i.e. deltaCD20 ou ΔCD20) est délété de tout ou partie de la partie transmembranaire du CD20 natif (ou wtCD20) ce qui ne permet pas l'ancrage du deltaCD20 à la membrane des lymphocytes B (Blood, vol. 112, no. 11, 16 novembre 2008, page 526).

Les demandeurs ont mis en évidence que l'expression du deltaCD20 était corrélée à la présence de certaines pathologies. De plus, il a également été mis en évidence que le niveau d'expression de deltaCD20 est corrélé à l'évolution de ces pathologies. Il s'agit plus particulièrement des pathologies liées à un disfonctionnement des lymphocytes B (également connues sous le nom d'hémopathies B) qui représentent 85 à 90% des hémopathies lymphoïdes. Il s'agit notamment des Lymphomes Folliculaires (LF), Leucémie lymphoïde chronique (LLC), Leucémie aigue lymphoblastique B (LAL), Lymphome du manteau (LM), Lymphomes B, Myélome, Maladie de Waldenström (MW).

Ainsi la présente invention concerne un kit de diagnostic comprenant une séquence identique à SEQ ID NO :14 ou SEQ ID NO :17. Selon un mode de réalisation tout à fait préféré, ladite sonde oligonucléotide à une séquence identique à SEQ ID NO : 14 ou à SEQ ID NO :17.

Dans le cadre de la présente invention, le terme « substantiellement identique » fait référence à deux séquences ayant plus de 90%, de préférence 95%, de manière encore plus préférée 99% et de façon tout à fait préférée 100% d'homologie.

Dans le cadre de la présente invention, le terme « polypeptide » fait référence à une séquence d'acides aminés comprenant ou ne comprenant pas de modifications post-traductionnelles. De manière préférée, le polypeptide selon l'invention est obtenu par synthèse ou par génie génétique, dans ce dernier cas, on parle de protéine recombinante.

Dans le cadre de la présente invention, le terme « séquence d'acides nucléiques » fait notamment référence aux séquences d'ADN ou d'ARN, naturels ou synthétiques. De préférence la séquence d'acides nucléiques selon l'invention est synthétique ou obtenue par génie génétique. Selon un autre mode de réalisation préféré, il s'agit d'une séquence d'acides nucléiques totalement ou partiellement purifiée.

Les vecteurs recombinants sont bien connus de l'homme du métier, ils permettent notamment la production de protéines recombinantes et/ou la multiplication d'une séquence d'acides nucléiques. De nombreux vecteurs recombinants sont disponibles dans l'art antérieur, on peut citer notamment les plasmides et les vecteurs viraux. Ainsi, selon un mode de réalisation préféré, le vecteur recombinant selon l'invention est choisi dans le groupe comprenant les plasmides et les vecteurs viraux.

Selon un premier mode de réalisation, le vecteur recombinant selon l'invention est un vecteur viral. Les vecteurs viraux sont bien connus de l'homme du métier et sont déjà utilisés en clinique chez l'homme (e.g. MVA, adénovirus, rétrovirus). Il s'agit la plupart du temps, d'un vecteur comprenant tout ou partie du génome d'un virus modifié pour intégrer une séquence exogène. Selon un mode de réalisation préféré, le vecteur viral selon l'invention est choisi dans le groupe comprenant les vecteurs adénoviraux, les vecteurs rétroviraux, les vecteurs poxviraux, les vecteurs dérivant des virus de l'herpes, les vecteurs dérivant des virus associés aux adénovirus et les vecteurs dérivant des alphavirus. La présente invention concerne également les particules virales comprenant les vecteurs recombinants selon l'invention.

De manière préférée, le vecteur recombinant selon l'invention est associé à un ou plusieurs composants facilitant son introduction dans une cellule hôte. Les composants facilitant l'introduction d'un vecteur dans une cellule hôte sont bien connus de l'homme du métier et certains sont disponibles commercialement, on parlera également d'agent de transfection. Dans un mode de réalisation tout à fait préféré, le composant facilitant l'introduction d'un vecteur recombinant selon l'invention dans une cellule hôte est choisi dans le groupe comprenant les lipides cationiques, les sels de calcium, les polymères cationiques et les polypeptides.

Dans le cadre de la présente demande, le terme « élément nécessaire à l'expression dans une cellule hôte » fait référence aux séquences d'acides nucléiques permettant la translation et la traduction d'une séquence d'acides nucléiques ainsi que les séquences d'acides nucléiques permettant d'augmenter lesdites traductions et translations. Selon un mode de réalisation préféré, l'élément nécessaire à l'expression dans une cellule hôte est choisi dans le groupe comprenant les introns, les sites de polyadénylation et les promoteurs.

Dans le cadre de la présente invention, le terme cellule hôte fait notamment référence aux cellules procaryotes et eucaryotes. Parmi ces cellules, on peut citer les bactéries, les levures, les cellules d'insectes (e.g. sf9) et les cellules animales (e.g. CHO, 293, PERC6). La présente invention concerne également une cellule hôte comprenant un vecteur recombinant selon l'invention.

Le terme « échantillon biologique » fait référence à tous les fluides ou tissus contenant les lymphocytes B du patient. Préférentiellement, l'échantillon biologique est le sang du patient ou la moelle osseuse.

Le procédé selon l'invention peut être mis en oeuvre avec un échantillon biologique brut (i.e. tel que prélevé sans avoir subi de modification), mais également après traitement de l'échantillon biologique par toutes les méthodes jugées souhaitables par l'homme du métier. Parmi ces traitements on peut citer la lyse des érythrocytes, l'isolement des cellules mononuclées (e.g. isolement sur Ficoll) et/ou l'ajout de molécules permettant la conservation de l'échantillon biologique (e.g. antiprotéases). Des techniques telles que la lyse des érythrocytes et l'isolement des cellules mononuclées permettent notamment d'augmenter la proportion de lymphocytes B par rapport aux autres cellules présentes dans l'échantillon biologique. Ainsi, selon un mode de réalisation préféré, le procédé de diagnostic selon l'invention comprend en outre une étape permettant d'augmenter la proportion des lymphocytes B par rapport aux autres cellules présentes dans l'échantillon biologique.

En ce qui concerne le polypeptide, il peut s'agir de la protéine avant ou après modifications post-traductionnelles et entière ou clivée. Le procédé concerne la détection de tout ou partie du polypeptide, ainsi il est possible de ne détecter qu'une partie de l'ARNm ou du polypeptide, pour autant que le niveau d'expression de cette partie soit représentatif de l'expression de la totalité de la molécule.

Dans la présente demande, le terme « niveau d'expression » fait référence à la quantité de deltaCD20 exprimée par les cellules. La mesure du niveau d'expression peut être faite de manière quantitative ou semi quantitative, en effet il n'est pas nécessaire de connaître la quantité exacte de deltaCD20, exprimée par les cellules, mais simplement de déterminer si cette quantité est significativement supérieure à une norme. Cette norme peut être facilement déterminée par l'homme du métier en utilisant un pool d'individus sains et en mesurant le niveau d'expression de deltaCD20 dans les cellules de ces individus. Si le procédé suivant l'invention révèle un niveau d'expression de deltaCD20 dans les cellules des patients supérieur au niveau d'expression normal, ledit patient sera susceptible d'être atteint d'un disfonctionnement des lymphocytes B.

Selon un mode de réalisation préféré, la mesure du niveau d'expression du polypeptide selon l'invention est la mesure du niveau d'expression de l'ARNm codant ledit polypeptide. Les techniques permettant de mesurer la quantité d'ARNm codant spécifiquement une molécule sont bien connues de l'homme du métier. Parmi ces techniques on peut citer la RT-PCR quantitative, la RT-PCR semi-quantitative, le northern blot et la technique des puces à ADN (en anglais « microarrays »). La construction et la production des sondes et des oligonucléotides nécessaires à la mise en oeuvre de ces techniques sont à la portée de l'homme du métier. Parmi ces oligonucléotides, on peut plus particulièrement citer ceux décrit par SEQ ID N0 :14 à SEQ ID N0 :17 qui sont également l'objet de la présente invention. Ainsi, selon un mode de réalisation encore plus préféré la mesure du niveau d'expression de l'ARNm codant polypeptide selon l'invention est faite par RT-PCR quantitative, RT-PCR semi-quantitative, RT-PCR quantitative en temps réel, northern blot ou par la technique des puces à ADN.

Selon un autre mode de réalisation préféré, ladite mesure du niveau d'expression d'un polypeptide selon l'invention et/ou d'un ARNm codant un polypeptide selon l'invention est la mesure du niveau d'expression d'un polypeptide selon l'invention et/ou d'un ARNm codant un polypeptide selon l'invention à l'intérieur des lymphocytes B.

Selon un mode de réalisation préféré, le procédé de diagnostic selon l'invention est un procédé de diagnostic d'une pathologie liée à un disfonctionnement d'un ou plusieurs lymphocytes B du patient. Selon un mode réalisation tout à fait préféré la pathologie liée à un disfonctionnement d'un ou plusieurs lymphocytes B est choisie dans le groupe comprenant les hémopathies B, les Lymphomes Folliculaires (LF), la Leucémie lymphoïde chronique (LLC), la Leucémie aigue lymphoblastique B (LAL), le Lymphome du manteau (LM), les Lymphomes B, le Myélome, et la Maladie de Waldenstrôm (MW).

En transplantation d'organes solides, incluant la transplantation rénale, la perte des greffons allogéniques reste un problème majeur. Le rôle des mécanismes des allo-anticorps du donneur dans le rejet hyper aigu est bien connu et partiellement maîtrisé, ainsi que les rejets précoces et tardifs reliés aux cellules T. Cependant les traitements affectant le compartiment lymphocytaire T ont un impact réduit sur la survie à long terme des greffons, suggérant d'autres mécanismes effecteurs cibles.

L'épissage du gène CD20 identifié, génère des transcrits délétés dont la séquence reste dans le cadre de lecture. La transcription de la forme épissée de l'ARNm ou la traduction en protéine pourrait interférer avec l'expression de la protéine normale CD20 et altérer son expression en surface, ce qui peut moduler l'efficacité du traitement au Rituximab. Cela a été récemment rapporté dans la littérature, ou l'acquisition de résistance à un anticorps anti-CD20 (Rituximab) dans des lignées cellulaires de lymphomes est associée à des mécanismes de régulation pré- et post-transcriptionnel ou des phénomènes épigénétiques. Ainsi, la protéine deltaCD20 constitue donc une nouvelle cible thérapeutique pour améliorer l'efficacité du traitement au Rituximab et prévenir les phénomènes d'échappement et de rechute.

En transplantation rénale, une étude de transcriptomique haut débit, chez des patients en rejet aigu a permis de définir un cluster de gènes caractéristique de la population de lymphocytes B, caractéristique de certains types de rejet aigus, non distinguable classiquement en microscopie optique. Des études complémentaires avec des techniques d'immunohistochimie ont confirmé une forte présence de Lymphocytes B CD20+ infiltrant le greffon. Enfin, il a été montré la persistance de cellules CD20+ infiltrant le greffon rénal post traitement au Rituximab, alors que le pool de Lymphocytes B circulant a été éliminé.

A la suite de ces travaux, des équipes ont décrits des études rapportant le traitement par Anticorps anti-CD20 (Rituximab) dans le cas de greffe rénale ou cardiaques chez des patients en rejet aigu précoces. Dans le cas de la plus large série de rejet aigu résistant au traitement par corticoïdes, ATG et plasmaphérèse, les résultats ont été encourageants avec 85% de survie des greffons à 2ans.

De manière générale, en transplantation d'organes solides, il y a un intérêt croissant pour le compartiment lymphocytaire B, impliqué dans la production d'allo-anticorps. Parmi les agents ciblant cette population de Lymphocytes B, le Rituximab montre tout naturellement son intérêt pour inhiber la réponse B, de manière préventive (pré transplantation) que de tardivement (ciblant les cellules B mémoires). L'utilisation du procédé de diagnostic selon l'invention (e.g. dans des fragments biopsiques ou sur échantillons urinaires non invasifs) et notre stratégie d'immunothérapie ciblant la protéine ΔCD20 sont utiles pour prédire un rejet aigu et pour améliorer le traitement au Rituximab.

Ainsi, Selon un mode réalisation tout à fait préféré la pathologie liée à un disfonctionnement d'un ou plusieurs lymphocytes B est le rejet aigu de greffes.

Ainsi, Selon un autre mode réalisation préféré le procédé de diagnostic selon l'invention est un procédé d'évaluation de l'efficacité d'un traitement comprenant l'utilisation d'un anticorps anti-CD20. Selon un mode de réalisation tout à fait préféré, ledit anticorps anti-CD20 est le Rituximab.

La présente demande concerne également un kit permettant de mettre en oeuvre le procédé de diagnostic selon l'invention.

La présente invention concerne également, l'utilisation d'un kit de diagnostic selon l'invention pour la détection d'une pathologie liée à un disfonctionnement d'un ou plusieurs lymphocytes B. Selon un mode de réalisation préféré, ladite pathologie liée à un disfonctionnement d'un ou plusieurs lymphocytes B est choisie dans le groupe comprenant les hémopathies B, les Lymphomes Folliculaires (LF), la Leucémie lymphoïde chronique (LLC), la Leucémie aigue lymphoblastique B (LAL), le Lymphome du manteau (LM), les Lymphomes B, le Myélome, et la Maladie de Waldenström (MW). Selon un autre mode de réalisation préféré, ladite pathologie liée à un disfonctionnement d'un ou plusieurs lymphocytes B est le rejet aigu de greffes.

La présente invention concerne également, l'utilisation d'un kit de diagnostic selon l'invention pour l'évaluation de l'efficacité d'un traitement comprenant l'utilisation d'un anticorps anti-CD20. Selon un mode de réalisation préféré, ledit anticorps anti-CD20 est le Rituximab.

Le delta CD20 est une cible idéale pour une stratégie d'immunothérapie (vaccination). Plus particulièrement, cette stratégie d'immunothérapie est utile dans le cadre des pathologies associées aux traitements utilisant un anticorps anti-CD20 (e.g. Rituximab) afin de prévenir les résistances et améliorer le traitement. En effet, les inventeurs ont montré en western blot, une augmentation du signal généré par la protéine tronquée lors de résistance au rituximab, induite in vitro (lignée B avec pression de sélection au Rituximab), corrélée avec l'augmentation du signal obtenu en RT-PCR quantitative à partir des ARNm des mêmes populations. Cela signifie que les cellules qui expriment le plus de deltaCD20 échappent et résistent au traitement standard par Rituximab. La protéine tronquée, deltaCD20 est une cible protéique potentielle, pour une réponse cellulaire cytotoxique T. De même, les ARNm splicés, décrit également sont une cible intéressante, par les technologies d'oligonucléotide antisens ou par des approches d'ARN silencing (SiRNA). Le but de ces 2 dernières approches est de permettre l'extinction in vivo de cet épissage alternatif.

La vaccination ou immunothérapie peptidique est une approche thérapeutique qui fait actuellement l'objet d'un grand intérêt dans le cadre de la prévention ou du traitement des cancers. Son principe repose sur l'immunisation par des peptides reproduisant des épitopes T d'antigènes tumoraux reconnus par les lymphocytes T cytotoxiques (CTL), qui jouent un rôle majeur dans l'élimination des cellules cancéreuses exprimant ces antigènes à leur surface.

Les CTL ne reconnaissent pas les antigènes protéiques entiers, mais des fragments peptidiques de ceux-ci, présentés par les molécules du complexe majeur d'histocompatibilité (CMH) exprimées à la surface de différentes cellules. Ce sont ces fragments peptidiques qui constituent les épitopes T.

La présentation de ces peptides résulte d'un processus complexe, dénommé « apprêtement de l'antigène », qui implique 3 étapes principales : 1/la dégradation cytosolique des antigènes par un complexe multienzymatique dénommé protéasome, 2/ la translocation des peptides issus de cette dégradation dans le réticulum endoplasmique (RE) par les transporteurs TAP, 3/ l'association de ces peptides avec le CMH pour former des complexes stables peptide/CMH, qui seront exportés à la surface cellulaire.

Les épitopes présentés par le complexe majeur d'histocompatibilité de classe I (CMH I) ont généralement 8 à 11 acides aminés, et sont reconnus par les cellules T CD8+, qui représentent la composante majeure de la réponse cytotoxique.

L'identification de ces épitopes, et notamment (compte tenu du rôle essentiel de la réponse CD8+ dans la cytotoxicité) de ceux présentés par le CMH I, constitue donc une étape essentielle pour le développement d'immunothérapies anti-tumorales.

De nombreux antigènes tumoraux capables d'induire une réponse CTL sont connus à l'heure actuelle. Certains des épitopes T de ces antigènes ont été identifiés, et l'efficacité de vaccins à base de peptides reproduisant ces épitopes T a été montrée dans de nombreux cas.

Suite à la découverte d'un nouvel épissage alternatif du gène CD20 humain (deltaCD20) et à la mise en évidence d'une protéine codée par cet ARNm tronqué, le demandeur a mis en évidence l'impact d'une telle cible en immunothérapie anti-tumorale. En effet, les résultats obtenus montrent un rôle de cette protéine cible (non exprimé dans des lymphocytes B des donneurs sains) dans l'oncogenèse ainsi que son implication dans les résistances au traitement par anticorps anti-CD20 (Rituximab).

La mise en évidence de la protéine deltaCD20 et l'induction d'une réponse cytotoxique T contre un peptide chevauchant la zone de jonction de l'épissage permet de mettre en oeuvre une immunothérapie par vaccination en complément des traitements standards (Anticorps anti-CD20 (Rituximab)) des pathologies liées à un disfonctionnement des lymphocytes B ou du rejet aigus de greffe. Cette vaccination peut se faire directement avec un peptide, ou par l'intermédiaire d'un vecteur recombinant codant ledit peptide.

Ainsi la présente invention concerne également l'utilisation d'un polypeptide tel que défini ci-dessous, d'une séquence d'acides nucléiques telle que définie ci-dessous, d'un vecteur recombinant comprenant la séquence d'acides nucléique telle que définie ci-dessous pour la préparation d'un médicament. Selon un mode de réalisation préféré, ledit polypeptide comprend au moins une séquence choisie parmi le groupe comprenant SEQ ID N0 : 6, SEQ ID N0 :7, SEQ ID N0 :8, SEQ ID N0 :9, SEQ ID N0 :10, SEQ ID N0 :11 SEQ ID N0 :12 et SEQ ID N0 :13. Selon un mode de réalisation encore plus préféré, ledit polypeptide consiste en un polypeptide ayant une séquence choisie parmi le groupe comprenant SEQ ID N0 :6, SEQ ID N0 :7, SEQ ID N0 :8, SEQ ID N0 :9, SEQ ID N0 :10, SEQ ID N0 :11 SEQ ID N0 :12 et SEQ ID N0 :13.

La présente invention concerne également, l'utilisation d'une séquence d'acides nucléiques codant un polypeptide comprenant au moins une séquence choisie parmi le groupe comprenant SEQ ID N0 :6, SEQ ID N0 :7, SEQ ID N0 :8, SEQ ID N0 :9, SEQ ID N0 :10, SEQ ID N0 :11 SEQ ID N0 :12 et SEQ ID N0 :13 pour la préparation d'un médicament. Selon un mode de réalisation préféré, l'utilisation d'une séquence d'acides nucléiques codant un polypeptide ayant une séquence consistant en une séquence choisie parmi le groupe comprenant SEQ ID N0 :6, SEQ ID N0 :7, SEQ ID N0 :8, SEQ ID N0 :9, SEQ ID N0 :10, SEQ ID N0 :11 SEQ ID N0 :12 et SEQ ID N0 :13 pour la préparation d'un médicament.

Selon un mode de réalisation encore plus préféré de ces utilisations pour la préparation d'un médicament, ledit polypeptide comprend au moins une séquence telle que SEQ ID N0 :9. Selon un mode de réalisation tout à fait préféré, L'utilisation selon l'invention est caractérisée en ce que ledit polypeptide consiste en un polypeptide ayant une séquence telle que SEQ ID N0 :9.

Selon un mode de réalisation préféré, ledit médicament est pour améliorer l'efficacité d'un traitement comprenant l'utilisation d'un anticorps anti-CD20. Selon un mode de réalisation encore plus préféré, ledit anticorps anti-CD20 est le Rituximab.

Selon un mode de réalisation préféré, ledit médicament pour le traitement ou la prévention des hémopathies B, des Lymphomes Folliculaires (LF), de la Leucémie lymphoïde chronique (LLC), de la Leucémie aigue lymphoblastique B (LAL), du Lymphome du manteau (LM), des Lymphomes B, du Myélome, et de la maladie de Waldenström (MW).

La présente invention concerne également, un polypeptide ayant une séquence choisie parmi le groupe comprenant SEQ ID N0 :6, SEQ ID N0 :7, SEQ ID N0 :8, SEQ ID N0 :9, SEQ ID N0 :10, SEQ ID N0 :11 SEQ ID N0 :12 et SEQ ID N0 :13.

Une autre approche, pourra être un transfert de gène, codant pour l'anticorps selon l'invention, redirigeant ainsi des lymphocytes T contre cette protéine. Le développement d'un anticorps monoclonal contre cette protéine permettra en parallèle d'identifier les séquences codant les régions hypervariables (CDR3) des chaînes légère et lourde des Ig. Les séquences identifiées pourront donc être transfectées à des cellules T par transfert de gène; le couplage avec un gène suicide pourra permettre de contrôler la réponse T anti-ΔCD20 quoiqu'elle se limitera à des T activés tumoraux exprimant cette forme de protéine. Ainsi, la présente invention concerne également l'utilisation d'un anticorps selon l'invention ou d'une séquence d'acides nucléiques codant ledit anticorps ou d'un vecteur comprenant ladite séquence d'acides nucléiques pour la préparation d'un médicament, et de manière préféré pour la préparation d'un médicament pour le traitement des pathologies dont la liste liées au disfonctionnement des lymphocytes B telles que décrites dans cette demande.

CD20, est à la fois un marqueur membranaire, puisqu'il est exprimé à la surface des cellules B, mais aussi et gène de « susceptibilité », puisqu'il code pour la molécule cible du traitement par anticorps anti-CD20 (Rituximab) dans certaines hémopathies. Ces 2 propriétés peuvent être mises à profit en thérapie génique, afin de modifier ex-vivo (e.g. par voie rétrovirale) ou in vivo des cellules T (n'exprimant normalement pas CD20). Ceci est notamment utile pour moduler et contrôler les complications post-allogreffe de moelle osseuse, dont sont responsables les lymphocytes T (LT). Ainsi, après modification génique, les cellules peuvent être sélectionnées (e.g. par un système immunomagnétique) sur la base de l'expression de CD20 (normalement pas exprimés par les LT) et peuvent être ciblées in vivo par anticorps anti-CD20 (Rituximab) en cas de survenue de complication (maladie du greffon contre l'hôte ou Graft versus Host Disease :GvHD).

Si l'intérêt d'utiliser le gène CD20 dans des protocoles de modifications géniques comme marqueur de sélection et comme gène de susceptibilité, différentes études ont montré une instabilité de l'expression de ce gène sous promoteur LTR de rétrovirus se caractérisant par une faible expression, incompatible avec son utilisation comme gène de susceptibilité. La production d'un épissage alternatif du gène CD20 peut être à l'origine de la modulation de l'expression. Nous avons d'ailleurs pu mettre en évidence la présence des transcrits alternatifs dans des lignées d'empaquetage des séquences rétrovirales transfectées avec un vecteur portant le cDNA CD20 « full length ». Cette lignée va donc générer des particules rétrovirales ΔCD20 qui pourront infecter des cellules cibles, mais sans expression de CD20 en surface, ce qui limitera les efficacités de la transduction rétrovirale. De plus, les cellules cibles transduites vont exprimer la forme épissée des transcrits CD20, qui peut altérer leur sensibilité au Rituximab.

Ainsi la découverte d'un épissage alternatif, et des sites donneurs et accepteurs relatifs à cette épissage, permet de produire une séquence d'acides nucléiques codant le CD20 natif et comprenant des modifications au niveau desdits sites donneurs et/ou accepteurs pour éviter ledit épissage alternatif. Ainsi, la présente invention concerne une séquence d'acide nucléique codant CD20 caractérisée en ce qu'elle ne comprend pas de site d'épissage alternatif. Selon un mode de réalisation préféré, ladite séquence comprend une séquence telle que SEQ ID N0 :5 et de façon tout à fait préféré, ladite séquence consiste en une séquence telle que SEQ ID N0 :5. La présente invention concerne également les vecteurs recombinants tels que définis précédemment comprenant ladite séquence d'acides nucléiques. La présente invention concerne également les particules virales comprenant lesdits vecteurs recombinants, lesdits vecteurs recombinants associés à un ou plusieurs composants facilitant leur introduction dans une cellule hôte telles que définis précédemment ainsi que les cellules hôtes (telles que définies précédemment) comprenant ledit vecteur recombinant. La présente invention, concerne également l'utilisation de ladite séquence d'acides nucléiques ou dudit vecteur recombinant pour la préparation d'un médicament, et préférentiellement pour la préparation d'un médicament pour améliorer l'efficacité d'un traitement comprenant l'utilisation d'un anticorps anti-CD20, et encore plus préférentiellement ledit anticorps anti-CD20 est le Rituximab.

La présente invention concerne également les compositions pharmaceutiques comprenant les polypeptides, les séquences d'acides nucléiques, les anticorps et/ou les oligonucléotides selon l'invention et un tampon pharmaceuticalement acceptable.

### EXPERIENCES

### 1. Mise en évidence de l'épissage alternatif du gène codant CD20.

Afin de cloner le cDNA codant pour CD20 dans un squelette plasmidique rétroviral, nous avons amplifié, par RT-PCR, à partir d'ARN extraits de la lignée B DAUDI, le segment correspondant à la totalité de la partie codante (CDS) de la protéine CD20, à l'aide de 2 amorces couvrant les codons « d'initiation ou start » et «d'arrêt ou stop » respectivement.

L'électrophorèse des produits de PCR a révélé 2 bandes distinctes, l'une de taille attendue de 894pb et l'autre de taille inférieure de 393pb et d'intensité identique.

Le séquençage et l'alignement sur NCBI Genebank a révélé une séquence correspondant au fragment codant du gène CD20, délété dans sa partie centrale de 501pb, avec conservation des codons « start » et « stop ». L'analyse de la délétion au niveau de la protéine montre que la partie transmembranaire est pratiquement totalement délétée ce qui est en défaveur d'un ancrage de cette protéine à la surface membranaire des cellules.

La protéine tronquée a une séquence de 131 acides aminés pour un poids moléculaire prédictif de 15 kD et elle inclu le domaine C-terminal intracytoplasmique, une petite partie du premier domaine transmembranaire et la fin du domaine N-terminal intracytoplasmique. La protéine tronquée potentielle n'est pas ancrée à la membrane par manque d'expression des 4 segments transmembranaires, comme nous avons pu le démontrer par imagerie en microscopie confocale, par transfection de lignées cellulaires avec un vecteur exprimant une protéine de fusion wtCD20/GFP ou ΔCD20/GFP. La protéine wtCD20Δ/GFP ou CD20/GFP ont été exprimée dans des cellules eucaryotes 293T après transfection avec un vecteur d'expression pcDNA3.1 CT topo (inVitrogen). La protéine GFP étant clonée dans le cadre de lecture de la séquence CD20 (Δ ou WT). La révélation de la protéine GFP a été réalisée par excitation directe (couleur verte) et la protéine wtCD20 par un anticorps antiCD20 couplée à TIRTC (couleur rouge). Ainsi, le marquage est cytoplasmique dans les cellules transfectées avec la forme tronquée de CD20 alors que l'on observe une colocalisation essentiellement membranaire des marques vert et rouge correspondant à la GFP et à wtCD20. Cela confirme que l'expression de ΔCD20 se limite au cytoplasme.

L'analyse de la séquence sauvage codante du gène CD20 (WTCD20) à l'aide de logiciels prédictifs des sites d'épissage Sites donneurs et accepteurs d'épissage (software NNSPLICE v0.9 - http://www.fruitfly.org/seq_tools/splice.htm) a montré la présence d'un site donneur (SD) et d'un site accepteur (SA) cryptique, correspondant exactement à la séquence codante du gène CD20 délétée (ΔCD20). Des recherches plus approfondies, avec d'autres suites logicielles (Netgene 2 - http://www.cbs.dtu.dk/services/NetGene2) ont permis de mettre en évidence un site de branchement, localisé à une vingtaine de paire de bases du site accepteur.

### 1. Expression de la protéine ΔCD20

### a. Détection par Western blot

A partir de lignées cellulaires d'origine B, nous avons recherché par western blot, avec des anticorps disponibles anti-CD20 reconnaissant la partie C-terminale de la protéine (Thermo Fischer, #RB9013), la présence d'une protéine tronquée (sans partie transmembranaire, donc cytoplasmique) codée par les transcrits « splicés ». Ainsi, nous avons pu montrer, en plus de la bande attendue correspondant à la protéine CD20 sauvage, la présence d'une bande de taille attendue (~17KD) correspondant à la protéine ΔCD20. Cette protéine n'est pas exprimée dans des lignées T.

### 2. ΔCD20 : biomarqueur moléculaire diagnostique et/ou pronostique des hémopathies B ou complication (Rejet aigu, lymphomes) post allogreffe de rein

### a. Transcrits alternatifs ΔCD20 et Hémopathies B

Nous avons criblé, avec un essai de PCR qualitatif, amplifiant la totalité de la séquence codante (flCD20 PCR) d'autre lignées dérivées d'hémopathies B de différents types (Burkitt, LAL pré-B, B-EBV transformée) et montré que la forme courte du transcrit CD20 était présente dans toutes les lignées B et absente dans des lignées T. Nous avons ensuite développé un outil de PCR sensible et spécifique (ΔCD20 PCR), par la conception d'une amorce couvrant le site de jonction de cet épissage. Ce nouvel outil permet de mettre en évidence uniquement la forme courte de l'ARNm et d'éviter ainsi les phénomènes de compétition en PCR, incompatible avec une grande sensibilité.

Disposant de cet outil de PCR plus performant, nous avons recherché la forme courte de CD20 sur des PBMC ou pour être plus sensible sur des cellules B CD19+ ou CD20+ de 5 donneurs sains sans pouvoir la détecter. La recherche spécifique du transcrit tronqué, grâce à notre outil sensible de PCR a montré un signal positif sur les lignées et un signal négatif sur les PBMC, suggérant la présence de ce mécanisme d'épissage dans des cellules transformées par EBV, dans des cellules malignes et l'absence dans des cellules B normales. Cette forme tronquée de l'ARN CD20 est donc la signature d'un état d'activation des cellules B associé à son phénotype malin.

### b. Quantification au diagnostique de ΔCD20 dans différents types d'hémopathies B

Afin d'illustrer la possibilité d'utiliser la quantification des transcrits ΔCD20 dans le diagnostiques de certaines hémopathies, nous avons appliqué notre essai de QRTPCR sur différents types d'hémopathies B. La quantification des 2 formes (full length et tronquée) à été réalisée contre une gamme de dilution en série, de quantité connue, de plasmides standards portant les formes wt ou Δde CD20. Le calcul du ratio R = [ΔCD20/(wtCD20+ΔCD20)] x 100 permet d'exprimer la quantité relative de transcrit CD20 par rapport à la forme sauvage. Nous avons ainsi pu montrer une expression différentielle de ces transcrits alternatifs dans différentes hémopathies B. Nous avons quantifié la forme épissée de ΔCD20 [exprimée en % of ΔCD20: R = (ΔCD20/wtCD20+ΔCD20) x 100] dans des lignées B-EBV transformées in vitro (2.9 ± 4.51%, n=6), dans des cellules CD19+ purifiées d'amygdalectomies (9 ± 2.2%, n=7) et de B-blastes produits in vitro (activation FLT3 ligand) (14 ± 7.8%, n=5). De manière intéressantes, nous avons quantifié ΔCD20 à 3.6 ± 5.1% dans des B-ALL (n=27), 3.9 ± 5.3% dans des lymphomes folliculaires (n=5); 2.9 ± 4.5% dans des lymphomes du manteau (n=6); 3.2 ± 2.2% dans des lymphomes de haut grade (n=5); et 0.1 ± 0.2% in B-CLL (n=8).

### c. ΔCD20 : Marqueur de maladie résiduelle (MRD)

Une autre possibilité serait également d'utiliser notre essai de quantification par PCR en temps réel pour suivre l'efficacité du traitement (chimiothérapie associée ou non au Rituximab - Rx-, par ex). Ce test est idéal pour des hémopathies ne présentant pas de marqueurs moléculaires ou phénotypiques caractéristiques. La cinétique de la quantification de ΔCD20 a été comparée avec l'expression de marqueurs moléculaires standards tels que les transcrits BCR/ABL (p190) et la cycline D1 respectivement dans le cas de LAL-B et du lymphome du manteau. Nous avons montré une corrélation entre les marqueurs usuels (cycline D1 et BCR/Abl p190) et ΔCD20.

### 3. La protéine ΔCD20, potentielle cible thérapeutique

### a. Résistance à un anticorps anti-CD20 (Rituximab)

Nous avons établi in vitro des résistances au Rx à partir de lignées B, en les exposant à des doses et des temps différents. Des tests de sensibilités in vitro, ont permis de vérifier l'acquisition de la résistance.

De manière très intéressante, nous avons montré par western blot une intensification du signal de la forme tronquée de la protéine CD20 en fonction de l'acquisition de la résistance au Rx de la population. Plus particulièrement, nous avons quantifié le mRNA codant CD20 dans les cellules de patients atteint de lymphomes folliculaires (n=3) ou de lymphome du manteau (n=3). Nous avons observé que l'acquisition de la résistance au Rituximab était, à chaque fois, corrélée à une augmentation de la quantité de mRNA codant CD20 (moyenne x3.38)

### b. Construction d'oligopeptides spécifiques de ΔCD20.

Ainsi, à partir de la traduction de la séquence de l'ARNm ΔCD20, nous avons utilisé la base SYFPEITHI (http://www.uni-tuebingen.de/uni/kxi/) pour dessiner des peptides chevauchant (AA30 à AA39) la zone de jonction de l'épissage alternatif et donc spécifiques de la forme ΔCD20). Grâce à un outil prédictif de clivage par le protéasome (http://paproc.de), 2 peptides (N°4-RMS et N°7-SLE) potentiellement immunogènes intéressant, car potentiellement préparés par les enzymes du protéoasome et restreints pour HLA-A*0201 ont été mis en évidence.

Les peptides correspondant à ces séquences ont été synthétisés par MILLEGEN (LABEGE, France), puis dissous dans du DMSO 20% et stockés à -80°C.

L'affinité de liaison des peptides ainsi identifiés pour la molécule du CMH concernée, ainsi que la stabilité du complexe peptide/molécule du CMH I a été vérifiée in vitro (données non montrée).

### c. Induction d'une réponse T cytotoxique par vaccination peptidique

L'immunogénicité des peptides ΔCD20a été évaluée par génération de CTL sur des souris transgéniques HLA-A2/DR1 et Knock-Out (KO) pour H-2 classe I et II.

Les souris (4 par groupe) sont immunisées avec des pools de 2 ou 3 peptides (2 injections à la base de la queue à 8 jours d'intervalle) avec : 50µg de chaque peptide, 100µg de peptide helper (peptide 20 mers issu de pp65 spécifique de CMV) l'ensemble co-émulsifié avec de l'adjuvent incomplet de Freud.

Sept jours après la 2ème immunisation, les rates des souris sont prélevées et les splénocytes sont re-stimulés in vitro avec 4µg/ml de chaque peptide séparément. Au 5ème jour de culture, les populations qui répondent sont testées pour déterminer une cytotoxicité spécifique. Les cellules, dont la cytotoxicité s'est avérée concluante, sont restimulées in vitro à des intervalles d'une semaine afin d'obtenir des CTL spécifiques.

Des cellules RMAS-HHD sont utilisées comme cibles pour étudier la cytotoxicité. Ces cellules-cibles sont chargées avec 10µg/ml du peptide à tester, ou d'un peptide témoin non-pertinent, à 37°C pendant 90 minutes puis marquées avec 100 µCi de 51Cr pendant 90 minutes, puis lavées trois fois. Les splénocytes sont étalés dans des plaques 96 puits à fond V (3×10 3 cellules/puits dans 100 µl de RPMI 1640 + 10% de sérum de veau foetal). Ensuite, 100 µl des cellules effectrices (rapport cellules effectrices/cellules cible = 30 :1 ; 10 :1 ; 3 :1 et 1 :1) sont ajoutés dans les puits et les plaques sont incubées à 37°C pendant 4 heures. Après incubation, 50 µl de surnageant sont collectés, transférés dans des plaques spécifiques (Lumaplate) et la radioactivité est mesurée dans un compteur y. Le pourcentage de lyse spécifique est calculé par la formule : [(libération de 51 Cr expérimentale - libération de 51 Cr spontanée) / (libération de 51 Cr maximale - libération de 51 Cr spontanée)] × 100.

Trois sur 4 souris de groupe d'immunisation A ont développé nettement une réponse cytotoxique T contre le peptide N°4 et pas de réponse contre un peptide tiers BMLF1, spécifique de EBV, indiquant ainsi la spécificité de la réponse.

Ces résultats montrent que l'immunisation par le peptide RMS génère des CTL qui tuent les cibles RMAS-HHD chargées avec ce même peptide, mais pas les cellules chargées avec le peptide non-pertinent.

Ces travaux montrent clairement que nous avons pu obtenir une réponse CTL anti-ΔCD20 qui pourra être utilisée pour améliorer le traitement par anticorps anti-CD20 et prévenir ou éliminer la persistance de lymphocytes B tumoraux CD20+ (exprimant la protéine ΔCD20).

### 4. Production par mutagénèse dirigé d'une séquence codant CD20 ne pouvant subir d'épissages alternatifs.

Nous avons donc entrepris de muter la séquence sauvage du gène CD20 humain, afin de limiter la formation de ces transcrits alternatifs. La mutation du site accepteur afin de modifier la séquence nucléotidique et conserver le cadre de lecture et donc la séquence amino-acidique - maintient de l'AA Gln, codé par le codon CAG ou CAA. La mutation a été réalisée au nucléotide 612 (coordonnée à partir de la première base du codon ATG-start) : -nt612G>A. Après mutagénèse dirigée, nous avons pu vérifier, par séquençage, la mutation d'une seule paire de base au niveau du site accepteur. Enfin, nous avons pu démontrer que la séquence du gène CD20 muté (mutCD20) ne générait plus de transcrit alternatif. Une analyse en cytométrie de flux de l'expression de CD20 prouve que l'expression est maintenue et que la protéine est effectivement exprimée à la membrane.

## Revendications

1. Un kit de diagnostic **caractérisé en ce qu'**il comprend au moins un oligonucléotide comprenant une séquence identique à SEQ ID NO : 14 ou SEQ ID NO :17.

2. Le kit de diagnostic selon la revendication 1 **caractérisé en ce qu'**il comprend au moins un oligonucléotide ayant une séquence identique à SEQ ID NO : 14 ou à SEQ ID NO :17.

3. Utilisation in vitro d'un kit de diagnostic selon l'une des revendications 1 à 2 pour la détection d'une pathologie liée à un disfonctionnement d'un ou plusieurs lymphocytes B.

4. L'utilisation in vitro d'un kit de diagnostic selon la revendication 3 **caractérisée en ce que** la pathologie liée à un disfonctionnement d'un ou plusieurs lymphocytes B est choisi dans le groupe comprenant les hémopathies B, les Lymphomes Folliculaires (LF), la Leucémie lymphoïde chronique (LLC), la Leucémie aigue lymphoblastique B (LAL), le Lymphome du manteau (LM), les Lymphomes B, le Myélome, et la Maladie de Waldenström (MW).

5. Utilisation in vitro d'un kit de diagnostic selon l'une des revendications 1 à 2 pour l'évaluation de l'efficacité d'un traitement comprenant l'utilisation d'un anticorps anti-CD20.

6. Utilisation in vitro d'un kit de diagnostic selon la revendication 5 **caractérisée en ce que** l'anticorps anti-CD20 est le Rituximab.

7. Utilisation d'un polypeptide comprenant au moins une séquence choisie parmi le groupe comprenant SEQ ID N0 :6, SEQ ID N0 :7, SEQ ID N0 :8, SEQ ID N0 :9, SEQ ID N0 :10, SEQ ID N0 :11 SEQ ID N0 :12 et SEQ ID N0 :13 pour la préparation d'un médicament.

8. Utilisation d'une séquence d'acides nucléiques codant un polypeptide comprenant au moins une séquence choisie parmi le groupe comprenant SEQ ID N0 :6, SEQ ID N0 :7, SEQ ID N0 :8, SEQ ID N0 :9, SEQ ID N0 :10, SEQ ID N0 :11 SEQ ID N0 :12 et SEQ ID N0 :13 pour la préparation d'un médicament.

9. Utilisation d'une séquence d'acides nucléiques codant un polypeptide ayant une séquence consistant en une séquence choisie parmi le groupe comprenant SEQ ID N0 :6, SEQ ID N0 :7, SEQ ID N0 :8, SEQ ID N0 :9, SEQ ID N0 :10, SEQ ID N0 :11 SEQ ID N0 :12 et SEQ ID N0 :13 pour la préparation d'un médicament.

10. L'utilisation selon la revendication 7 ou 8 **caractérisée en ce que** ledit polypeptide comprend au moins une séquence telle que SEQ ID N0 :9.

11. L'utilisation selon la revendication 10 **caractérisée en ce que** ledit polypeptide consiste en un polypeptide ayant une séquence telle que SEQ ID N0 :9.

12. Utilisations selon l'une des revendications 7 à 11 pour améliorer l'efficacité d'un traitement comprenant l'utilisation d'un anticorps anti-CD20.

13. Utilisation selon la revendication 12 **caractérisée en ce que** l'anticorps anti-CD20 est le Rituximab.

14. Un polypeptide ayant une séquence choisie parmi le groupe comprenant SEQ ID N0 :6, SEQ ID N0 :7, SEQ ID N0 :8, SEQ ID N0 :9, SEQ ID N0 :10, SEQ ID N0 :11 SEQ ID N0 :12 et SEQ ID N0 :13.

15. Une séquence d'acide nucléique codant CD20 **caractérisée en ce qu'**elle comprend une séquence telle que SEQ ID N0 :5.

16. La séquence selon la revendication 15 **caractérisée en ce qu'**elle consiste en une séquence telle que SEQ ID N0 :5.

17. Un vecteur recombinant comprenant une séquence d'acides nucléiques selon l'une des revendications 15 à 16 placée sous le contrôle d'un ou plusieurs éléments nécessaires à son expression dans une cellule hôte.

18. Le vecteur recombinant selon la revendication 17 **caractérisé en ce qu'**il est choisi dans le groupe comprenant les plasmides et les vecteurs viraux.

19. Le vecteur recombinant selon la revendication 18 **caractérisé en ce qu'**il est un vecteur viral choisi dans le groupe comprenant les vecteurs adénoviraux, les vecteurs rétroviraux, les vecteurs poxviraux, les vecteurs dérivant des virus de l'herpes, les vecteurs dérivant des virus associés aux adénovirus et les vecteurs dérivant des alphavirus.

20. Le vecteur recombinant selon la revendication 18 **caractérisé en ce qu'**il est associé à un ou plusieurs composants facilitant son introduction dans une cellule hôte.

21. Le vecteur recombinant selon la revendication 20 **caractérisé en ce que** le composant facilitant son introduction dans une cellule hôte est choisi dans le groupe comprenant les lipides cationiques, les sels de calcium, les polymères cationiques et les polypeptides.

22. Le vecteur recombinant selon l'une des revendications 18 à 21 **caractérisé en ce que** l'élément nécessaire à son expression dans une cellule hôte est choisi dans le groupe comprenant les introns, les sites de polyadénylation et les promoteurs.

23. Utilisation d'une séquence d'acides nucléiques selon l'une des revendications 15 à 16 ou d'un vecteur recombinant selon l'une des revendications 17 à 22 pour la préparation d'un médicament.

24. Utilisations selon la revendication 23 pour améliorer l'efficacité d'un traitement comprenant l'utilisation d'un anticorps anti-CD20.

25. Utilisation selon la revendication 24 **caractérisée en ce que** l'anticorps anti-CD20 est le Rituximab.

## Patentansprüche

1. Diagnose-Kit, **dadurch gekennzeichnet, dass** es mindestens ein Oligonukleotid beinhaltet, das eine Sequenz aufweist, die identisch ist zu SEQ ID NO: 14 oder SEQ ID NO : 17.

2. Diagnose-Kit nach Anspruch 1, **dadurch gekennzeichnet, dass** es mindestens ein Oligonukleotid beinhaltet, das eine Sequenz aufweist, die identisch ist zu SEQ ID NO : 14 oder SEQ ID NO : 17.

3. In-vitro-Verwendung eines Diagnose-Kit nach einem der Ansprüche 1 bis 2 für die Erkennung einer Pathologie, die mit einer Funktionsstörung eines oder mehrerer B-Lymphozyten in Verbindung steht.

4. In-vitro-Verwendung eines Diagnose-Kit nach Anspruch 3, **dadurch gekennzeichnet, dass** die Pathologie, die mit einer Funktionsstörung eines oder mehreren B-Lymphozyten in Verbindung steht, aus der Gruppe gewählt ist, die beinhaltet: B-Hämopathien, Follikel-Lymphome (LF), chronische lymphatische Leukämie (LLC), akute B-lymphoblastische Leukämie (LAL), Mantelzellenlymphom (LM), B-Lymphome, ein Myelom und Morbus Waldenström (MW).

5. In-vitro-Verwendung eines Diagnose-Kit nach einem der Ansprüche 1 bis 2 für die Bewertung der Effizienz einer Behandlung, welche die Verwendung eines anti-CD20-Antikörpers beinhaltet.

6. In-vitro-Verwendung eines Diagnose-Kit nach Anspruch 5, **dadurch gekennzeichnet, dass** der anti-CD20-Antikörper Rituximab ist.

7. Verwendung eines Polypeptids, aufweisend mindestens eine Sequenz, die aus der Gruppe gewählt ist, welche SEQ ID NO : 6, SEQ ID NO : 7, SEQ ID NO : 8, SEQ ID NO : 9, SEQ ID NO : 10, SEQ ID NO : 11, SEQ ID NO : 12 und SEQ ID NO : 13 beinhaltet, und zwar für die Herstellung eines Medikaments.

8. Verwendung einer Nukleinsäurensequenz, die ein Polypeptid codiert, aufweisend mindestens eine Sequenz, die aus der Gruppe gewählt ist, welche SEQ ID NO : 6, SEQ ID NO : 7, SEQ ID NO : 8, SEQ ID NO : 9, SEQ ID NO : 10, SEQ ID NO : 11, SEQ ID NO : 12 und SEQ ID NO : 13 beinhaltet, und zwar für die Herstellung eines Medikaments.

9. Verwendung einer Nukleinsäurensequenz, die ein Polypeptid codiert, bestehend aus einer Sequenz, die aus der Gruppe gewählt ist, welche SEQ ID NO : 6, SEQ ID NO : 7, SEQ ID NO : 8, SEQ ID NO : 9, SEQ ID NO : 10, SEQ ID NO : 11, SEQ ID NO : 12 und SEQ ID NO : 13 beinhaltet, und zwar für die Herstellung eines Medikaments.

10. Verwendung nach Anspruch 7 oder 8, **dadurch gekennzeichnet, dass** das Polypeptid mindestens eine Sequenz wie SEQ ID NO : 9 aufweist.

11. Verwendung nach Anspruch 10, **dadurch gekennzeichnet, dass** das Polypeptid aus einem Polypeptid besteht, das eine Sequenz wie SEQ ID NO : 9 aufweist.

12. Verwendungen nach einem der Ansprüche 7 bis 11 zur Verbesserung der Effizienz einer Behandlung, welche die Verwendung eines anti-CD20-Antikörpers beinhaltet.

13. Verwendung nach Anspruch 12, **dadurch gekennzeichnet, dass** der anti-CD20-Antikörper Rituximab ist.

14. Polypeptid, das eine Sequenz aufweist, die aus der Gruppe gewählt ist, die SEQ ID NO : 6, SEQ ID NO : 7, SEQ ID NO : 8, SEQ ID NO : 9, SEQ ID NO : 10, SEQ ID NO : 11, SEQ ID NO : 12 und SEQ ID NO : 13 beinhaltet.

15. Nukleinsäuresequenz, die CD20 codiert, **dadurch gekennzeichnet, dass** sie eine Sequenz wie SEQ ID NO : 5 aufweist.

16. Sequenz nach Anspruch 15, **dadurch gekennzeichnet, dass** sie aus einer Sequenz wie SEQ ID NO : 5 besteht.

17. Rekombinanter Vektor, der eine Nukleinsäuresequenz nach einem der Ansprüche 15 bis 16 aufweist, die unter der Kontrolle eines oder mehrerer Elemente, die für deren Expression erforderlich sind, in eine Wirtszelle eingesetzt wird.

18. Rekombinanter Vektor nach Anspruch 17, **dadurch gekennzeichnet, dass** er aus der Gruppe gewählt ist, welche Plasmide und virale Vektoren beinhaltet.

19. Rekombinanter Vektor nach Anspruch 18, **dadurch gekennzeichnet, dass** er ein viraler Vektor ist, der aus der Gruppe gewählt ist, welche beinhaltet: adenovirale Vektoren, retrovirale Vektoren, poxvirale Vektoren, Vektoren, die von Herpesviren stammen, Vektoren, die von zu Adenoviren gehörenden Viren stammen, und Vektoren, die von Alphaviren stammen.

20. Rekombinanter Vektor nach Anspruch 18, **dadurch gekennzeichnet, dass** er mit einem oder mehreren Bestandteilen assoziiert ist, die dessen Einbringen in eine Wirtszelle erleichtern.

21. Rekombinanter Vektor nach Anspruch 20, **dadurch gekennzeichnet, dass** der Bestandteil, der dessen Einbringen in eine Wirtszelle erleichtert, aus der Gruppe gewählt ist, welche kationische Lipide, Calciumsalze, kationische Polymere und Polypeptide beinhaltet.

22. Rekombinanter Vektor nach einem der Ansprüche 18 bis 21, **dadurch gekennzeichnet, dass** das Element, das zu dessen Expression in einer Wirtszelle erforderlich ist, aus der Gruppe gewählt ist, welche Introns, Polyadenylierungsorte und Promotoren beinhaltet.

23. Verwendung einer Nukleinsäuresequenz nach einem der Ansprüche 15 bis 16 oder eines rekombinanten Vektors nach einem der Ansprüche 17 bis 22 für die Herstellung eines Medikaments.

24. Verwendungen nach Anspruch 23 zur Verbesserung der Effizienz einer Behandlung, welche die Verwendung eines anti-CD20-Antikörpers beinhaltet.

25. Verwendung nach Anspruch 24, **dadurch gekennzeichnet, dass** der anti-CD20-Antikörper Rituximab ist.

## Claims

1. Diagnostic kit **characterised in that** it comprises at least one oligonucleotide comprising a sequence identical to SEQ ID NO: 14 or SEQ ID NO: 17.

2. Diagnostic kit according to claim 1 **characterised in that** it comprises at least one oligonucleotide having a sequence identical to SEQ ID NO: 14 or SEQ ID NO: 17.

3. In vitro use of a diagnostic kit according to any of claims 1 to 2 for detecting a disease associated with dysfunction of one or a plurality of B lymphocytes.

4. In vitro use of a diagnostic kit according to claim 3 **characterised in that** the disease associated with dysfunction of one or a plurality of B lymphocytes is selected in the group comprising Follicular Lymphomas (FL), Chronic lymphocytic leukaemia (CLL), B-cell acute lymphoblastic leukaemia (ALL), mantle cell lymphoma (ML), B-cell lymphomas, Myeloma and Waldenström's Disease (WD).

5. In vitro use of a diagnostic kit according to any of claims 1 to 2 for evaluating the efficacy of a treatment comprising the use of an anti-CD20 antibody.

6. In vitro use of a diagnostic kit according to claim 5 **characterised in that** the anti-CD20 antibody is Rituximab.

7. Use of a polypeptide comprising at least one sequence selected from the group comprising SEQ ID NO: 6, SEQ ID NO: 7, SEQ ID NO: 8, SEQ ID NO: 9, SEQ ID NO: 10, SEQ ID NO: 11, SEQ ID NO: 12 and SEQ ID NO: 13 for preparing a drug.

8. Use of a nucleic acid sequence encoding a polypeptide comprising at least one sequence selected from the group comprising SEQ ID NO: 6, SEQ ID NO: 7, SEQ ID NO: 8, SEQ ID NO: 9, SEQ ID NO: 10, SEQ ID NO: 11, SEQ ID NO: 12 and SEQ ID NO: 13 for preparing a drug.

9. Use of a nucleic acid sequence encoding a polypeptide having a sequence consisting of a sequence selected from the group comprising SEQ ID NO: 6, SEQ ID NO: 7, SEQ ID NO: 8, SEQ ID NO: 9, SEQ ID NO: 10, SEQ ID NO: 11, SEQ ID NO: 12 and SEQ ID NO: 13 for preparing a drug.

10. Use according to claim 7 or 8 **characterised in that** said polypeptide comprises at least one sequence such as SEQ ID NO: 9.

11. Use according to claim 10 **characterised in that** said polypeptide consists of a polypeptide having a sequence such as SEQ ID NO: 9.

12. Uses according to any of claims 7 to 11 for improving the efficacy of a treatment comprising the use of an anti-CD20 antibody.

13. Use according to claim 12 **characterised in that** the anti-CD20 antibody is Rituximab.

14. Polypeptide having a sequence selected from the group comprising SEQ ID NO: 6, SEQ ID NO: 7, SEQ ID NO: 8, SEQ ID NO: 9, SEQ ID NO: 10, SEQ ID NO: 11, SEQ ID NO: 12 and SEQ ID NO: 13.

15. Nucleic acid sequence encoding CD20 **characterised in that** it comprises a sequence such as SEQ ID NO: 5.

16. Sequence according to claim 15 **characterised in that** it consists of a sequence such as SEQ ID NO: 5.

17. Recombinant vector comprising a nucleic acid sequence according to any of claims 15 to 16 placed under the control of one or a plurality of elements required for the expression thereof in a host cell.

18. Recombinant vector according to claim 17 **characterised in that** it is selected in the group comprising plasmids and viral vectors.

19. Recombinant vector according to claim 18 **characterised in that** it is a viral vector selected in the group comprising adenovirus vectors, retrovirus vectors, poxvirus vectors, herpes virus-derived vectors, vectors derived from viruses associated with adenoviruses and alphavirus-derived vectors.

20. Recombinant vector according to claim 18 **characterised in that** it is associated with one or a plurality of compounds facilitating the introduction thereof into a host cell.

21. Recombinant vector according to claim 20 **characterised in that** the compound facilitating the introduction thereof into a host cell is selected in the group comprising cationic lipids, calcium salts, cationic polymers and polypeptides.

22. Recombinant vector according to any of claims 18 to 21 **characterised in that** the element required for expression in a host cell is selected in the group comprising introns, polyadenylation sites and promoters.

23. Use of a nucleic acid sequence according to any of claims 15 to 16 or a recombinant vector according to any of claims 17 to 22 for preparing a drug.

24. Uses according to claim 23 for improving the efficacy of a treatment comprising the use of an anti-CD20 antibody.

25. Use according to claim 24 **characterised in that** the anti-CD20 antibody is Rituximab.
